# EUROPÄISCHE PATENTSCHRIFT

(11) **EP 2 521 708 B1**
(45) Veröffentlichungstag und Bekanntmachung des Hinweises auf die Patenterteilung: **01.01.2014**
(21) Anmeldenummer: 11700322.8
(22) Anmeldetag: 03.01.2011
(51) Int. Cl.: C07C 57/07, C07C 67/48, C07C 67/54, G01F 1/36, G01F 1/40

(54) **VERFAHREN ZUM FÖRDERN EINES STROMS EINER (METH)ACRYLMONOMERE ENTHALTENDEN FLÜSSIGKEIT**
METHOD FOR DELIVERING A FLOW OF A LIQUID CONTAINING (METH)ACRYLIC MONOMERS
PROCÉDÉ DE TRANSPORT D'UN FLUX DE LIQUIDE CONTENANT DES MONOMÈRES (MÉTH)ACRYLIQUES

(30) Priorität: 06.01.2010 US 292509 P; 06.01.2010 DE 102010000706
(43) Veröffentlichungstag der Anmeldung: 14.11.2012
(73) Patentinhaber: BASF SE, 67056 Ludwigshafen (DE)
(72) Erfinder: RISSEL, Steffen, 67281 Kirchheim (DE); SCHMIDT, Oswin, 67158 Ellerstadt (DE)
(86) Internationale Anmeldenummer: PCT/EP2011/050023
(87) Internationale Veröffentlichungsnummer: WO 2011/083104

(56) Entgegenhaltungen:
- WO-A1-2009/021921
- GB-A- 2 042 187

## Beschreibung

Vorliegende Erfindung betrifft ein Verfahren zum Fördern eines Stroms einer (Meth)acrylmonomere enthaltenden Flüssigkeit F mittels einer Förderpumpe P durch eine Rohrleitung hindurch, deren offene, für den Strom der Flüssigkeit F durchlässige Querschnittsfläche QF kreisförmig ist und in der an einer in Förderrichtung des Stroms der Flüssigkeit F hinter und/oder vor der Förderpumpe P gelegenen Stelle zum Zweck der Messung und/oder zum Zweck der Begrenzung der Stärke des durch die Rohrleitung geförderten Stroms der Flüssigkeit F eine Blende B angebracht ist, die dort eine Teilfläche der offenen kreisförmigen Querschnittsfläche QF schließt, so dass an dieser Stelle nur noch eine offene Restfläche RF der Querschnittsfläche QF für den Strom der Flüssigkeit F zum Hindurchströmen verbleibt.

Die Schreibweise (Meth)acrylmonomere steht in dieser Schrift verkürzend für "Acrylmonomere und/oder Methacrylmonomere". Der Betriff Acrylmonomere steht in dieser Schrift verkürzend für Acrylsäure, Ester der Acrylsäure und/oder Acrylnitril. Der Begriff Methacrylmonomere steht in dieser Schrift verkürzend für Methacrylsäure, Ester der Methacrylsäure und/oder Methacrylnitril.

Zu den vorgenannten Estern gehören unter anderem Ester aus Acrylsäure und einem 1 bis 12 C-Atome aufweisenden Alkohol sowie Ester aus Methacrylsäure und einem 1 bis 12 C-Atome aufweisenden Alkohol. Als Alkohole kommen dabei sowohl einwertige (weisen eine -OH-Gruppe auf) als auch mehrwertige (weisen mehr als eine -OH-Gruppe auf) Alkohole in Betracht. Zu diesen Alkoholen gehören insbesondere 1 bis 12, vorzugsweise 1 bis 8 C-Atome aufweisende ein- und mehrwertige Alkohole.

Diese Definition beinhaltet nicht in notwendiger Weise, dass die Herstellung dieser Ester durch Reaktion der entsprechenden Alkohole mit der jeweiligen Säure erfolgen muss. Vielmehr kommen als Herstellverfahren auch andere Reaktionen wie z.B. Umesterungen oder Additionsreaktionen in Betracht.

Im Besonderen sollen in dieser Schrift die nachfolgenden (Meth)acrylsäureester unter dem Begriff (Meth)acrylmonomere subsumieren: Hydroxyethylacrylat, Hydroxyethylmethacrylat, Hydroxypropylacrylat, Hydroxypropylmethacrylat, Glycydylacrylat, Glycydylmethacrylat, Methylacrylat, Methylmethacrylat, n-Butylacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, 2-Propylheptylacrylat, N,N-Dimethylaminoethylacrylat und N,N-Dimethylaminoethylmethacrylat.

Ferner subsumieren unter dem Begriff "(Meth)acrylmonomere" die Monomere Acrolein und Methacrolein, die insbesondere als Zwischenprodukte (z.B. für die Herstellung von Acrylsäure und Methacrylsäure) von Bedeutung sind.

Im Übrigen sind (Meth)acrylmonomere (insbesondere die individuell genannten) wichtige Ausgangsverbindungen zur Herstellung von Polymerisaten, die z.B. als Klebstoffe Verwendung finden.

(Meth)acrolein sowie (Meth)acrylsäure werden großtechnisch überwiegend durch heterogen katalysierte partielle Gasphasenoxidation geeigneter C₃-/C₄-Vorläuferverbindungen, insbesondere von Propen und Propan im Fall von Acrolein (einstufige Partialoxidation) und Acrylsäure (einstufige oder zweistufige Partialoxidation mit Acrolein als Zwischenprodukt) bzw. von iso-Buten und iso-Butan im Fall des Methacrolein (einstufige Partialoxidation) und der Methacrylsäure (einstufige oder zweistufige Partialoxidation mit Methacrolein als Zwischenprodukt), hergestellt. Neben Propen, Propan, iso-Buten und iso-Butan eignen sich als Ausgangsstoffe jedoch auch andere 3 bzw. 4 Kohlenstoffatome enthaltende Verbindungen, beispielsweise iso-Butanol, n-Propanol oder der Methylether von iso-Butanol.

Dabei wird normalerweise ein Produktgasgemisch erhalten, aus dem das (Meth)acrolein bzw. die (Meth)acrylsäure durch absorptive, rektifikative, extraktive und/oder kristallisative Verfahren abgetrennt werden muss (vgl. z.B. DE-A 10 224 341). In entsprechender Weise ist (Meth)acrylnitril durch katalytische Ammoxidation von vorgenannten C₃-/C₄-Vorläuferverbindungen und nachfolgende Abtrennung aus dem Produktgasgemisch erhältlich.

Ester der (Meth)acrylsäure sind z.B. durch direkte Umsetzung von (Meth)acrylsäure mit den entsprechenden Alkoholen erhältlich. Allerdings fallen auch in diesem Fall zunächst Produktgemische an, aus denen die (Meth)acrylsäureester z.B. rektifikativ und/oder extraktiv abgetrennt werden müssen.

Insbesondere im Zusammenhang mit den vorgenannten Abtrennungen ist es immer wieder erforderlich, (Meth)acrylmonomere in mehr oder weniger reiner Form oder in Lösung befindlich zu fördern (in dieser Schrift generell als (Meth)acrylmonomere enthaltende Flüssigkeiten F bezeichnet).

Das Lösungsmittel kann dabei sowohl wässrig als auch ein organisches Lösungsmittel sein. Die spezifische Art des Lösungsmittels ist erfindungsgemäß im Wesentlichen unbeachtlich. Selbstredend können auch die (Meth)acrylmonomeren selbst das Lösungsmittel und Verunreinigungen (Nebenkomponenten) das Gelöste bilden. Der Gehalt von zu fördernden Flüssigkeiten F (insbesondere Lösungen) an (Meth)acrylmonomeren kann z.B. ≥ 5 Gew.-%, oder ≥ 10 Gew.-%, oder ≥ 20 Gew.-%, oder ≥ 40 Gew.-%, oder ≥ 60 Gew.-%, oder ≥ 80 Gew.-%, oder ≥ 90 Gew.-%, oder ≥ 95 Gew.-%, oder ≥ 99 Gew.-% betragen (insbesondere auch im Rahmen der erfindungsgemäßen Verfahrensweise).

Üblicherweise erfolgt die Förderung einer (Meth)acrylmonomere enthaltenden Flüssigkeit F mittels einer Förderpumpe P (vgl. z.B. DE-A 10 228 859) als ein durch eine Rohrleitung hindurch fließender Strom derselben.

Dabei weist das Innere der Rohrleitung normalterweise eine kreisförmige offene Querschnittsfläche QF auf, durch die hindurch der Strom der Flüssigkeit F fließt. Mit dem Begriff "Förderpumpe P" sind dabei ebenso wie stets in dieser Schrift Pumpen zur Förderung von Flüssigkeiten (d.h., im Wesentlichen inkompressiblen Medien) bezeichnet. Sie weisen in der Regel eine Saugseite und eine Druckseite auf. Durch eine mit der Saugseite verbundene Rohrleitung saugt die Förderpumpe P den Strom der zu fördernden Flüssigkeit dabei an. In der Förderpumpe P wird die zu fördernde Flüssigkeit anschließend auf einen höheren Druck gebracht und durch eine mit ihrer Druckseite verbundene Rohrleitung in die gewünschte Förderrichtung weggedrückt. Zur Förderung von (Meth)acrylmonomere enthaltenden Flüssigkeiten F eigenen sich dabei insbesondere die in der DE-A 10 228 859 und in der DE-A 10 2008 054 587 beschriebenen Förderpumpen (insbesondere die in diesen Schriften beschriebenen Radialkreiselpumpen).

Insbesondere im stationären Betrieb einer Herstellung von (Meth)acrylmonomeren ist es normalerweise von Bedeutung, dass die Förderung eines Stroms einer (Meth)acrylmonomere enthaltenden Flüssigkeit F mittels einer Förderpumpe P durch eine Rohrleitung hindurch mit einer über die Betriebszeit im Wesentlichen konstanten Stärke des Stroms der Flüssigkeit F erfolgt. Schwankungen der Stromstärke (die z.B. durch Schwankungen von Produktionsbedingungen im Herstellungsprozess verursacht sein können) kann z.B. dadurch entgegen gewirkt werden, dass der Zugang zur Saugseite bzw. Druckseite der jeweiligen Förderpumpe P über entsprechende Armaturen variabel geöffnet oder gesperrt werden kann. D.h., die Einstellung der erforderlichen Förderstromstärke kann über eine Anpassung der Öffnung der Armatur erfolgen. Häufig handelt es sich bei der Förderpumpe P anwendungstechnisch vorteilhaft jedoch um eine "Drehzahl"-geregelte Förderpumpe. In diesem Fall erfolgt die Einstellung der erforderlichen Förderstromstärke nicht über eine Anpassung des freien Querschnitts in der Armatur, sondern über eine Anpassung der Drehzahl der Förderpumpe. Selbstverständlich können beide Arten der Stromstärkeeinstellung auch kombiniert angewendet werden.

Bevor eine Nachstellung der Förderstromstärke vorgenommen werden kann, bedarf es jedoch eines Messverfahrens, das eine Abweichung der Stärke des durch die Rohrleitung geförderten Stroms der Flüssigkeit F von ihrem Sollwert feststellt.

Zur Durchflussmessung strömender Flüssigkeiten sind verschiedene Messverfahren entwickelt worden. Man unterscheidet dabei Verfahren zur Messung des Volumen- und des Massenstroms. Bei Kenntnis der Massendichte der geförderten Flüssigkeit F sind beide ineinander umrechenbar.

Ein anwendungstechnisch besonders vorteilhaftes Verfahren zur Messung des Volumenstroms einer in einer Rohrleitung geförderten Flüssigkeit F ist das sogenannte Wirkdruckverfahren. Bei diesem Verfahren erzeugt ein in der Rohrleitung angebrachter Wirkdruckgeber einen Differenzdruck (Wirkdruck), den ein angeschlossenes Gerät (Durchflussmesser oder Differenzdruck-Messumformer) erfasst. Der Wirkdruckgeber ist normalerweise ein in die Rohrleitung eingebrachter Strömungswiderstand und der Wirkdruck ist die Differenz zwischen dem in Strömungsrichtung unmittelbar vor dem Strömungswiderstand und dem in Strömungsrichtung unmittelbar hinter dem Strömungswiderstand herrschenden Druck. Die Übertragung der beiden Drucke kann z.B. über in der Rohrleitung angebrachte Bohrungen und mit selbigen verbundene Wirkdruckleitungen bis zum Wirkdruckempfänger erfolgen, der z.B. ein Differenzdruckmanometer sein kann, welches unmittelbar den Wirkdruck anzeigt. Selbiger wird dann über die Bernoulli-Gleichung mit dem Volumenstrom in Verbindung gebracht.

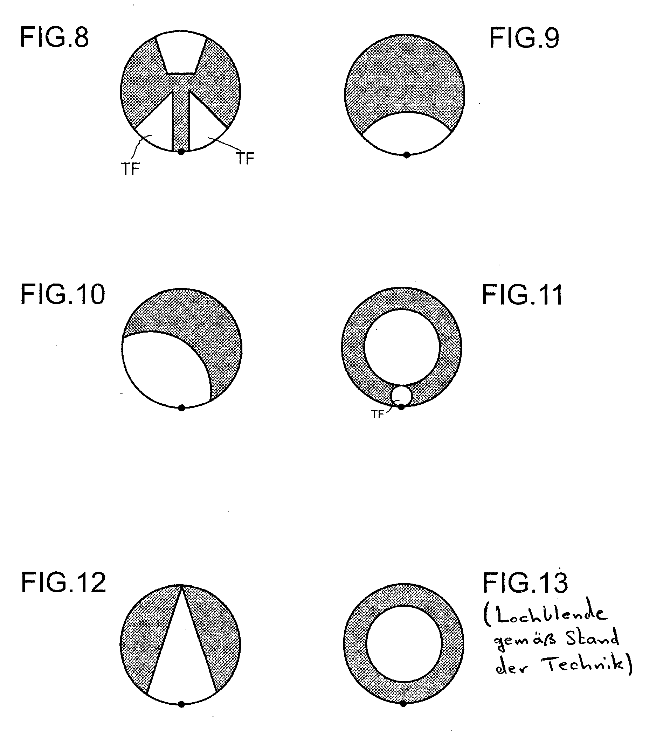

Anwendungstechnisch zweckmäßig wird als Wirkdruckgeber eine Lochblende verwendet. Im Prinzip handelt es sich bei einer Lochblende um eine Blechscheibe (z.B. aus Edelstahlblech), mit einer kreisförmigen Öffnung in der Mitte (siehe Figur 13). Durch ihren Einbau in die Rohrleitung wird der Innendurchmesser der Rohrleitung an der Einbaustelle von einem größeren Wert auf einen geringeren Wert verjüngt und so eine Drosselung des Durchflusses eines Stroms einer Flüssigkeit F bewirkt. Die für den Strom der Flüssigkeit F durchlässige Querschnittsfläche ist an der Stelle, an der die Lochblende in der Rohrleitung eingebracht ist, jedoch nach wie vor kreisförmig, wobei das Kreiszentrum auf der Symmetrieachse der Rohrleitung liegt.

Lochblenden werden aber nicht nur zum Zweck der Messung der Stärke eines durch eine Rohrleitung geförderten Stroms einer Flüssigkeit F in eine Rohrleitung eingebracht. Häufig verfolgt der Einbau einer Lochblende in eine Rohrleitung vielmehr den Zweck, die Stärke des durch eine Rohrleitung geförderten Stroms, bezogen auf denselben Förderdruck, zu begrenzen. Der zum Beibehalt der Förderleistung erforderliche erhöhte Förderdruck ermöglicht so bei einer in Förderrichtung der Flüssigkeit F in der Rohrleitung angebrachten Lochblende (auch Ringblende) in der vorab der Lochblende befindlichen Förderstrecke z.B. die Einstellung eines erhöhten Drucks. Letzterer erlaubt z.B. eine vergleichsweise weitergehende Erhöhung der Temperatur der geförderten Flüssigkeit F, ohne dass mit der Temperaturerhöhung ein Sieden der geförderten Flüssigkeit F einhergeht. Von diesem Grundprinzip wird z.B. im Rahmen des Einsatzes von Zwangsumlaufentspannungsverdampfern Gebrauch gemacht, wie es beispielhaft in der EP-A 854 129 und in der DE-A 102008054587 sowie in der DE-A 102009027401 ausgeführt ist.

Eine andere Zielsetzung eines Verfahrens der Herstellung von (Meth)acrylmonomeren besteht üblicherweise darin, das Verfahren über eine möglichst lange Betriebsdauer ungestört betreiben zu können. Als eine Ursache für eine von Zeit zu Zeit erforderliche Unterbrechung der Verfahren zur Herstellung von (Meth)acrylmonomeren haben sich Verschlüsse von Rohrleitungen erwiesen, in welchen (Meth)acrylmonomere enthaltende Flüssigkeiten F mittels einer Förderpumpe P gefördert werden. Dies insbesondere dann, wenn in die Rohrleitung zum Zweck der Messung und/oder zum Zweck der Begrenzung der Stärke des durch die Rohrleitung geförderten Stroms einer (Meth)acrylmonomere enthaltenden Flüssigkeit F eine Lochblende angebracht ist.

Die Aufgabe der vorliegenden Erfindung bestand darin, den vorstehend beschriebenen Nachteil wenigstens zu mindern.

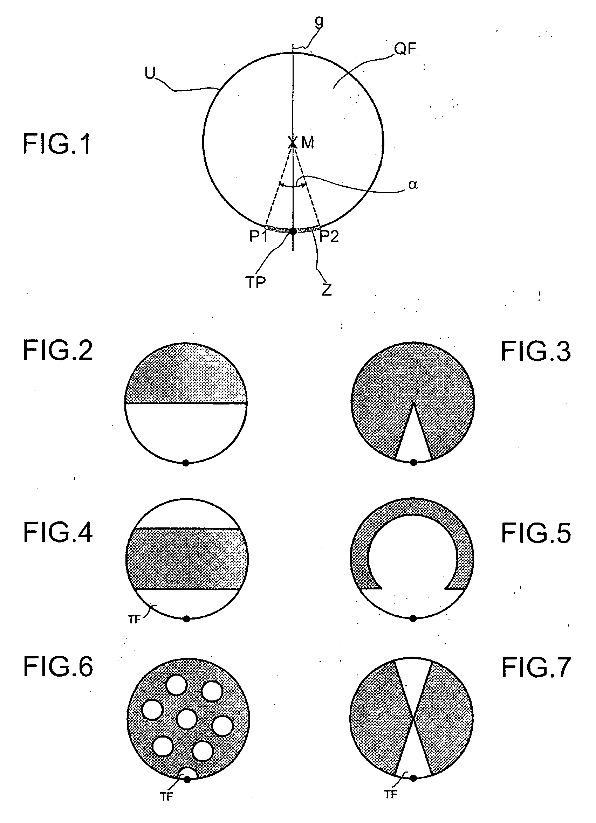

Demgemäß wird ein Verfahren zum Fördern eines Stroms einer (Meth)acrylmonomere enthaltenden Flüssigkeit F mittels einer Förderpumpe P durch eine Rohrleitung hindurch, deren offene, für den Strom der Flüssigkeit F durchlässige Querschnittsfläche QF kreisförmig ist und in der an einer in Förderrichtung des Stroms der Flüssigkeit F hinter und/oder vor der Förderpumpe P gelegenen Stelle zum Zweck der Messung und/oder zum Zweck der Begrenzung der Stärke des durch die Rohrleitung geförderten Stroms der Flüssigkeit F eine Blende B angebracht ist, die dort eine Teilfläche der offenen kreisförmigen Querschnittsfläche QF schließt, so dass an dieser Stelle nur noch eine offene Restfläche RF der Querschnittsfläche QF (für den Strom der Flüssigkeit F zum Hindurchströmen) verbleibt, zur Verfügung gestellt, das dadurch gekennzeichnet ist, dass die in der Rohrleitung angebrachte Blende B als Bestandteil der offenen Restfläche RF eine Teilfläche TF der kreisförmigen Querschnittsfläche QF offen lässt, deren Umrisslinie einen Kreisbogen der kreisförmigen Umrisslinie der Querschnittsfläche QF (in dieser Schrift auch als Umrisslinie U bezeichnet) umfasst, welcher im unteren Zehntel der kreisförmigen Umrisslinie der Querschnittsfläche QF liegt. Die Flüssigkeit F kann dabei sowohl eine Lösung (optisch homogenes, in der Regel transparentes System) als auch ein heterogenes Gemisch unterschiedlicher Phasen (z.B. Gas- und Flüssigkeitsphase, vorzugsweise jedoch keine Festphase) sein. Legt man auf dem Umfang eines Kreises zwei beliebige Punkte fest und verbindet sie durch die kürzest mögliche auf dem Kreisumfang verlaufende Kreislinie, so wird diese die beiden Punkte verbindende Kreislinie in dieser Schrift als Kreisbogen bezeichnet. Das untere Zehntel der kreisförmigen Umrisslinie der Querschnittsfläche QF meint den Kreisbogen Z (auf dem der Punkt TP liegt) zwischen den beiden Punkten P₁ und P₂ auf dem Kreisumfang (der kreisförmigen Umrisslinie U), die mit dem Mittelpunkt M der kreisförmigen Querschnittsfläche QF ein gleichschenkliges Dreieck bilden, bei welchem der Eckwinkel α mit dem Mittelpunkt M als zugehörigem Eckpunkt 36° beträgt und wobei die Winkelhalbierende dieses Eckwinkels die durch den Mittelpunkt M und durch den tiefstgetegenen Punkt der kreisförmigen Querschnittsfläche QF verlaufende Gerade g ist (vgl. Figur 1). Erfindungsgemäß vorteilhaft umfasst die Umrisslinie der Teilfläche TF wenigstens 5 %, oder wenigstens 10 %, oder wenigstens 20 %, vorzugsweise wenigstens 30 %, besonders bevorzugt wenigstens 40 %, mit Vorteil wenigstens 50 %, besser wenigstens 60 % oder wenigstens 70 %. noch besser wenigstens 80 % oder wenigstens 90 % und am Besten 100 % des unteren Zehntels der kreisförmigen Umrisslinie der Querschnittsfläche QF.

Die WO 2009/021921 A1 offenbart ein Verfahren zur Herstellung wasserabsorbierender Harze, wobei eine wässrige Acrylsäurelösung in eine Pipeline eingespeist und durch die Pipeline zu einem Acrylsäure-Verarbeitungsort geleitet wird. Eine Blende ist in der Rohrleitung nicht angebracht.

Die GB 2042187 A beschreibt (siehe Figur 1) eine Rohrleitung mit einer Blende, die jedoch keine Blende mit einem Loch im unteren Zehntel der Umrisslinie ist.

Mit besonderem Vorteil umfasst die Umrisslinie der Teilfläche TF dabei einen Kreisbogen der kreisförmigen Umrisslinie der Querschnittsfläche QF, auf welchem der (geometrisch) am tiefsten gelegene Punkt TP der kreisförmigen Umrisslinie der Quer schnittsfläche QF liegt.

Erfindungsgemäße Verfahren sind demnach insbesondere solche Verfahren zum Fördern eines Stroms einer (Meth)acrylmonomere enthaltenden Flüssigkeit F mittels einer Förderpumpe P durch eine Rohrleitung hindurch, deren offene, für den Strom der Flüssigkeit F durchlässige Querschnittsfläche QF kreisförmig ist und in der an einer in Förderrichtung des Stroms der Flüssigkeit F hinter und/oder vor der Förderpumpe P gelegenen Stelle zum Zweck der Messung und/oder zum Zweck der Begrenzung der Stärke des durch die Rohrleitung geförderten Stroms der Flüssigkeit F eine Blende B angebracht ist, die dort eine Teilfläche der offenen kreisförmigen Querschnittsfläche QF schließt, so dass an dieser Stelle nur noch eine offene Restfläche RF der Querschnittsfläche QF verbleibt, die dadurch gekennzeichnet sind, dass die in der Rohrleitung angebrachte Blende B als Bestandteil der offenen Restfläche RF eine Teilfläche TF der kreisförmigen Querschnittsfläche QF offen lässt, deren Umrisslinie einen Kreisbogen der kreisförmigen Umrisslinie der Querschnittsfläche QF umfasst, welcher im unteren Zwanzigstel der kreisförmigen Umrisslinie der Querschnittsfläche QF liegt.

Das untere Zwanzigstel der kreisförmigen Umrisslinie der Querschnittsfläche QF meint dabei den Kreisbogen Z* (auf dem der Punkt TP liegt) zwischen den beiden Punkten P₃ und P₄ auf dem Kreisumfang (der kreisförmigen Umrisslinie U), die mit dem Mittelpunkt M der kreisförmigen Querschnittsfläche QF ein gleichschenkliges Dreieck bilden, bei welchem der Eckwinkel α* mit dem Mittelpunkt M als zugehörigem Eckpunkt 18° beträgt und wobei die Winkelhalbierende dieses Eckwinkels die durch den Mittelpunkt M und durch den tiefstgelegenen Punkt der kreisförmigen Querschnittsfläche QF verlaufende Gerade g ist.

Bevorzugt umfasst die Umrisslinie der Teilfläche TF dabei wenigsten 20 %. vorzugsweise wenigstens 30 %, besonders bevorzugt wenigstens 40 %, mit Vorteil wenigstens 50 %, besser wenigstens 60 % oder wenigstens 70 %, noch besser wenigstens 80 % oder wenigstens 90 % und am Besten 100 % des unteren Zwanzigstels der kreisförmigen Umrisslinie der Querschnittsfläche QF.

Mit besonderem Vorteil umfasst die Umrisslinie der Teilfläche TF auch hierbei einen Kreisbogen der kreisförmigen Umrisslinie der Querschnittsfläche QF, auf welchem der (geometrisch) am tiefsten gelegene Punkt TP der kreisförmigen Umrisslinie der Querschnittsfläche QF liegt.

Prinzipiell kann die offene Restfläche RF beim erfindungsgemäßen Verfahren wenigstens 10%, oder wenigstens 20%, oder wenigstens 30%, oder wenigstens 40%, oder wenigstens 50%, oder wenigstens 60%, oder wenigstens 70%, oder wenigstens 80%, oder wenigstens 90% der Querschnittsfläche QF betragen. In der Regel wird die offene Restfläche RF beim erfindungsgemäßen Verfahren jedoch ≤ 90%, häufig ≤ 80%, vielfach ≤ 70%, oft ≤ 60% und teilweise ≤ 50% der Querschnittsfläche QF betragen. Die Querschnittsfläche QF meint dabei wie stets in dieser Schrift die kreisförmige Querschnittsfläche an derjenigen Stelle in der Rohrleitung, an der die Blende B angebracht ist.

Ferner kann die Teilfläche TF beim erfindungsgemäßen Verfahren mit der offenen Restfläche RF identisch sein. Selbstredend kann sich die Teilfläche TF beim erfindungsgemäßen Verfahren aber auch nur über einen Teil (z.B. 5 bis 95%, oder 10 bis 90%, oder 20 bis 80%, oder 30 bis 70%, oder 40 bis 60%) der Restfläche RF erstrecken.

Verschiedene Ausführungsformen von erfindungsgemäß geeigneten in die Rohrleitung eingebrachten Blenden B zeigen die Figuren 2 bis 12. Die dunkle Fläche zeigt dabei die Draufsicht (Vorderansicht) auf die Blende B. Die helle Fläche zeigt dabei die Draufsicht auf die offene Restfläche RF sowie (als wenigstens Teilfläche derselben) die jeweilige offene Teilfläche TF. Außerdem zeigen die Figuren jeweils den am tiefsten gelegenen Punkt TP.

Figur 1 zeigt in einer schematischen Darstellung die kreisförmige Querschnittsfläche QF, die zugehörige Umrisslinie U derselben, die Punkte P₁ und P₂ auf dem Kreisumfang, den Mittelpunkt M der kreisförmigen Querschnittsfläche QF, den Winkel α = 36°, den am tiefsten gelegenen Punkt TP der kreisförmigen Umrisslinie U der kreisförmigen Querschnittsfläche QF sowie das untere Zehntel Z der kreisförmigen Umrisslinie U der Querschnittsfläche QF. Figur 13 zeigt eine Lochblende gemäß Stand der Technik. Ihre Verwendung im Stand der Technik ist insbesondere auf ihre hohe Symmetrie zurückzuführen. Anwendungstechnisch zweckmäßig ist die dunkle Fläche in den Figuren 2 bis 12 als in den Querschnitt der Rohrleitung eingeschweißte Stahlbleche (z.B. Edelstahlbleche des DIN-Typs 1.4539 oder 1.4571) verwirklicht. Die Dicke des Stahlblechs beträgt typisch 2 bis 15, häufig 5 bis 10 mm.

Selbstverständlich kann die Umrisslinie der Teilfläche TF einen Kreisbogen der kreisförmigen Umrisslinie der Querschnittsfläche QF umfassen, der z. B. 100% der unteren Hälfte der kreisförmigen Umrisslinie der Querschnittsfläche QF entspricht (vgl. z.B. Fig. 2).

Die Rohrleitung in welcher die erfindungsgemäße Förderung einer (Meth)acryl-monomere enthaltenden Flüssigkeit F erfolgt, muss keinen über ihre Gesamtlänge einheitlichen Rohrquerschnitt aufweisen. Auch kann sie Abschnitte aufweisen, in denen ihr Querschnitt nicht kreisförmig ist. Erfindungsgemäß wesentlich ist jedoch, dass ihr Querschnitt an derjenigen Stelle, an der die Blende B in die Rohrleitung eingebaut ist, kreisförmig ist und den Flächeninhalt QF aufweist. Eine Blende B kann dabei erfindungsgemäß in der zur Saugseite der Förderpumpe P führenden Rohrleitung und/oder in der von der Druckseite der Förderpumpe P wegführenden Rohrleitung eingebaut sein.

Der zur kreisförmigen Querschnittsfläche QF gehörige Innendurchmesser der Rohrleitung, in welcher die Förderung einer (Meth)acrylmonomere enthaltenden Flüssigkeit erfolgt, kann 1 bis 100, oft 10 bis 90 oder 20 bis 80 bzw. 30 bis 70 oder 40 bis 60 cm betragen. Erfindungsgemäß bevorzugt wird als Förderpumpe P eine Kreiselpumpe (insbesondere eine Radialkreiselpumpe) eingesetzt, wie sie die DE-A 10228859 und die DE-A 102008054587 empfehlen.

Das erfindungsgemäße Verfahren eignet sich im Fall aller Flüssigkeiten F, die wenigstens ein in dieser Schrift genanntes (Meth)acrylmonomeres enthalten.

Normalerweise enthält eine erfindungsgemäß geförderte Flüssigkeit F radikalische Polymerisationsinhibitoren zugesetzt, die einer unerwünschten radikalischen Polymerisation der in der Flüssigkeit F enthaltenen (Meth)acrylmonomere entgegenwirken. Eine solche unerwünschte radikalische Polymerisation kann z.B. durch Temperatur, Licht oder sonstige spontan ausgelöste Radikalbildung erfolgen. Bevorzugt verwendete Polymerisationsinhibitoren für erfindungsgemäß zu fördernde Flüssigkeiten F sind z.B. p-Methoxyphenol (MEHQ), Phenothiazin (PTZ), Hydrochinon, Phenol (z.B. 2,4-Dimethyl-6,6-butylpehnol), Chinone, Butylbrenzkatechin, Diphenylamin, p-Phenylendiamine, Nitroxyl-Radikale (z.B. 4-Hydroxy-2,2,6,6-tetramethylpiperidin-1-oxyl (4-OH-TEMPO)) und/oder Nitrosoverbindungen wie z.B. Nitrophenole (sowie alle anderen in der WO 00/64947 genannten Polymerisationsinhibitoren). Typische Polymersationsinhibitorgehalte erfindungsgemäß zu fördernder Flüssigkeiten F betragen 50 bis 2000 Gew.-ppm, häufig 100 bis 1000 Gew.-ppm, jeweils bezogen auf das Gewicht der Flüssigkeit F.

Die Vorteilhaftigkeit der erfindungsgemäßen Verfahrensweise gegenüber einer Verwendung von Lochblenden B ist vermutlich darauf zurückzuführen, dass erfindungsgemäß zu fördernde (Meth)acrylmonomere enthaltende Flüssigkeiten F selbst im Beisein von in ihnen gelöst enthaltenen Polymerisationsinhibitoren in geringen Mengen unerwünschtes radikalisches Polymerisat und/oder Oligomerisat der (Meth)acrylmonomere gelöst enthalten. Der Wortlaut "gelöst" meint dabei sowohl "molekular gelöst" als auch "kolloidal gelöst". Beides aber nur insoweit, als die gelöste Form in der zu fördernden Flüssigkeit visuell (d.h. mit bloßem menschlichem Auge) nicht wahrnehmbar ist.

Beim Fördern einer (Meth)acrylmonomere enthaltenden Flüssigkeit (z.B. Lösung) F durch die offene Restfläche der Querschnittsfläche QF hindurch, die beim Einbau einer Lochblende verbleibt, kommt es nun zu einem Aufrahmen des in der Flüssigkeit F molekular und/oder kolloidal gelöst enthaltenen ungewollten Polymerisats und/oder Oligomerisats der (Meth)acrylmonomeren. Dies ist darauf zurückzuführen, dass der die Lochöffnung der Lochblende umgebende Ring der Blende für die Flüssigkeit F undurchlässig ist. Das Polymerisat und/oder Oligomerisat reichert sich so im Lauf der Zeit in der in Strömungsrichtung im Nahbereich vor der Lochblende befindlichen Raum enthaltenen Flüssigkeit F an (es kommt zur Ausbildung von Polymerisat und/oder Oligomerisat angereichert enthaltendem "Rahm" der Flüssigkeit F). Aus eigenen Untersuchungen ist aber bekannt, dass insbesondere in Flüssigkeiten F gelöst enthaltenes Oligomerisat und Polymerisat (im Unterschied zu makroskopisch sichtbarem Polymerisat) eine ausgeprägte polymerisationsfördernde Wirkung auf (Meth)acrylmonomere ausübt (insbesondere im Fall von Acrylsäure). Dies gilt vor allem bei auf atomaren Wasserstoff bezogenen relativen Molekulargewichten von ≥ 1000, bzw. ≥ 2000, oder ≥ 3000 des gelösten Polymerisats und/oder Oligomerisats. Im Lauf der Betriebsdauer stellt sich so mit erhöhter Wahrscheinlichkeit ein Verschluss der Lochöffnung der Lochblende ein, der eine Verfahrensunterbrechung erforderlich macht. In der Regel bildet sich ein solcher Verschluss aber auch mit höherer Geschwindigkeit aus.

Bei einer erfindungsgemäßen Verfahrensweise erfolgt dagegen parallel zur Rahmbildung an der durch die Blende B verschlossenen Teilfläche der Querschnittsfläche QF kontinuierlich eine Entrahmung aufgrund von Rahmabfluß durch die offene Teilfläche TF hindurch. Bei gleicher Größe der Restfläche RF resultiert daraus eine verminderte Verschlussneigung.

Wie bereits erwähnt, ist die erfindungsgemäße Verfahrensweise nicht nur im Zusammenhang mit der Bestimmung (Messung) der Stärke des durch eine Rohrleitung geförderten Stroms einer (Meth)acrylmonomere enthaltenden Flüssigkeit F von Bedeutung.

Vielmehr ist die erfindungsgemäße Verfahrensweise vor allem auch als Element einer Zufuhr von thermischer Energie in eine (Meth)acrylmonomere enthaltende Flüssigkeit F von Bedeutung, die dadurch praktiziert wird, dass man die Flüssigkeit F durch eine Zwangsumlaufentspannungsvorrichtung fördert (vgl. EP-A 854129), die mittels einer Blende B gedrosselt wird.

Somit umfasst die vorliegende Erfindung insbesondere auch ein Verfahren der kontinuierlichen thermischen Auftrennung wenigstens eines Stroms eines (Meth)acrylmonomere enthaltenden Gemischs G in einer thermischen Trennvorrichtung, die
- eine Förderpumpe P, die eine Saugseite und eine Druckseite aufweist,
- einen indirekten Umlaufwärmeaustauscher UW, der einen Sekundärraum S und einen von dem Sekundärraum S durch eine materielle Trennwand T getrennten Primärraum PR umfasst, der einen Eingang E und einen Ausgang A aufweist,
- einen eine Zuführstelle ZU und eine Rückführstelle RT aufweisenden trennwirksamen Raum R mit oder ohne trennwirksame Einbauten, in den an der Zuführstelle ZU kontinuierlich der Strom des Gemischs G hineingeführt wird,
- eine erste Förderverbindung F1, die vom trennwirksamen Raum R zur Saugseite der Förderpumpe P führt,
- eine zweite Förderverbindung F2, die von der Druckseite der Förderpumpe P zum Eingang E in den Primärraum PR des Umlaufwärmeaustauschers UW führt,
- eine dritte Förderverbindung F3, die vom Ausgang A aus dem Primärraum PR des Umlaufwärmeaustauschers UW zu der Rückführstelle RT in den trennwirksamen Raum R zurückführt und eine Rohrleitung RL mit einer offenen kreisförmigen Querschnittsfläche QF aufweist, und
- eine Blende B, die in der Rohrleitung RL der Förderverbindung F3 an einer zwischen dem Ausgang A aus dem Primärraum PR des Umlaufwärmeaustauschers UW und der Rückführstelle RT in den trennwirksamen Raum R gelegenen Stelle angebracht ist und dort eine Teilfläche der offenen kreisförmigen Querschnittsfläche QF der Rohrleitung RL schließt, so dass an dieser Stelle nur noch eine offene Restfläche RF der Querschnittsfläche QF in der Rohrleitung RL der Förderverbindung F3 verbleibt,
umfasst, und bei dem wenigstens ein Teil der für die thermische Auftrennung des Gemischs G im trennwirksamen Raum R erforderlichen Energie dadurch in denselben eingebracht wird, dass
- die Förderpumpe P aus dem trennwirksamen Raum R kontinuierlich einen flüssigen, die Temperatur T^{SI} aufweisenden, (Meth)acrylmonomere enthaltenden Stoffstrom ST ansaugt,
- die Förderpumpe P den von ihr angesaugten Stoffstrom ST durch die Förderverbindung F2, den Primärraum PR des Umlaufwärmeaustauschers UW sowie die Förderverbindung F3 hindurch an der Rückführstelle RT in den trennwirksamen Raum R zurückpumpt, und
- gleichzeitig durch den Sekundärraum S ein fluider Wärmeträger WT, dessen Temperatur T^{WT}, mit der er in den Sekundärraum S hineingeführt wird, größer als die Temperatur T^{SE} des Stoffstroms ST am Eingang E in den Primärraum PR ist, so hindurchgeführt wird, dass der Stoffstrom ST aus dem Ausgang A des Primärraums PR des Umlaufwärmeaustauschers UW flüssig auf die Restfläche RF zuströmt und die Restfläche RF mit der Temperatur T^{SII} > T^{SI} durchströmt,
das dadurch gekennzeichnet ist, dass die in der Rohrleitung RL der Förderverbindung F3 eingebaute Blende B als Bestandteil der offenen Restfläche RF eine Teilfläche TF der kreisförmigen Querschnittsfläche QF offen lässt, deren Umrisslinie einen Kreisbogen der kreisförmigen Umrisslinie der Querschnittsfläche QF umfasst, welcher im unteren Zehntel der kreisförmigen Umrisslinie der Querschnittsfläche QF liegt.

Erfindungsgemäß vorteilhaft umfasst auch hier die Umrisslinie der Teilfläche TF wenigstens 20 %, vorzugsweise wenigstens 30 %, besonders bevorzugt wenigstens 40 %, mit Vorteil wenigstens 50 %, besser wenigstens 60 %, oder wenigstens 70 %, noch besser wenigstens 80 %, oder wenigstens 90 % und am Besten 100 % des unteren Zehntels der kreisförmigen Umrisslinie der Querschnittsfläche QF.

Mit besonderem Vorteil umfasst die Umrisslinie der Teilfläche TF dabei auch hier einen Kreisbogen der kreisförmigen Umrisslinie der Querschnittsfläche QF, auf welchem der (geometrisch) am tiefsten gelegene Punkt TP der kreisförmigen Umrisslinie liegt.

Erfindungsgemäße Verfahren der kontinuierlichen thermischen Auftrennung wenigstens eines Stroms eines (Meth)acrylmonomere enthaltenden Gemischs G in einer thermischen Trennvorrichtung der vorstehend beschriebenen Art sind daher insbesondere solche, die dadurch gekennzeichnet sind, dass die in der Rohrleitung der Förderverbindung F3 eingebaute Blende als Bestandteil der offenen Restfläche RF eine Teilfläche TF der kreisförmigen Querschnittsfläche QF offen lässt, deren Umrisslinie einen Kreisbogen der kreisförmigen Umrisslinie der Querschnittsfläche QF umfasst, welcher im unteren Zwanzigstel der kreisförmigen Umrisslinie der Querschnittsfläche QF liegt.

Erfindungsgemäß vorteilhaft umfasst auch in diesem Fall die Umrisslinie der Teilfläche TF wenigstens 20 %, vorzugsweise wenigstens 30 %, besonders bevorzugt wenigstens 40 %, mit Vorteil wenigstens 50 %, besser wenigstens 60 %, oder wenigstens 70 %, noch besser wenigstens 80 %, oder wenigstens 90 % und am Besten 100 % des unteren Zwanzigstels der kreisförmigen Umrisslinie der Querschnittsfläche QF.

Mit besonderem Vorteil umfasst die Umrisslinie der Teilfläche TF auch hierbei einen Kreisbogen der kreisförmigen Umrisslinie der Querschnittsfläche QF, auf welchem der (geometrisch) am tiefsten gelegene Punkt TP der kreisförmigen Umrisslinie der Querschnittsfläche QF liegt.

Im Übrigen gelten alle zum erfindungsgemäßen Verfahren des Förderns eines Stroms einer (Meth)acrylmonomere enthaltenden Flüssigkeit F mittels einer Förderpumpe P durch eine mit einer Blende B ausgerüsteten Rohrleitung mit der Querschnittsfläche QF hindurch getroffenen Aussagen in entsprechender Weise.

Normalerweise weist der trennwirksame Raum R neben der Zuführstelle ZU und der Rückführstelle RT noch wenigstens einen Auslass AU1, aus dem ein (Meth)acrylmonomere angereichert enthaltender Stoffstrom 1, und wenigstens einen Auslass AU2, aus dem ein (Meth)acrylmonomere abgereichert enthaltender Stoffstrom 2 herausgeführt wird, auf.

Bei den Förderverbindungen F1, F2 und F3 kann es sich unabhängig voneinander im einfachsten Fall jeweils um eine Rohrleitung handeln. Grundsätzlich kann eine solche Förderverbindung aber auch aus einer Kombination von Rohrleitungen oder auch aus einer Verbindung von Rohrleitungen mit Schläuchen oder Förderschächten mit z.B. rechteckigem Querschnitt bestehen.

Grundsätzlich kann das Gemisch G bei den relevanten Verfahren der thermischen Auftrennung an der Zuführstelle ZU gasförmig in den trennwirksamen Raum R geführt werden. In der Regel wird das Gemisch G an der Zuführstelle ZU jedoch flüssig in den trennwirksamen Raum R geführt. Häufig erfolgt diese Zufuhr jedoch auch als ein Gemisch aus gasförmiger und flüssiger Phase. Charakteristisch für die relevanten Verfahren der thermischen Auftrennung ist, dass die mit ihnen erzielte Trennwirkung die Zufuhr von thermischer Energie erfordert. Die Erzielung der thermischen Trennwirkung selbst kann prinzipiell in trennwirksamen Räumen erzielt werden, die keine trennwirksamen Einbauten umfassen, wie es z. B. bei einer einfachen Destillation der Fall ist (z.B. eine leere Kolonne). Dabei wird ein flüssiges Gemisch partiell verdampft und die dabei erzeugte, eine andere Zusammensetzung als das flüssige Gemisch aufweisende Dampfphase in dampfförmiger und/oder in kondensierter Form abgetrennt. Häufig wird die thermische Trennwirkung jedoch unter Mitwirkung trennwirksamer Einbauten erzielt, wobei in der Regel gasförmige (meist aufsteigend) und flüssige (meist absteigend) Stoffströme im trennwirksamen Raum (z. B. eine Einbauten enthaltende Trennkolonne) im Gleich- oder Gegenstrom geführt werden. Infolge des zwischen den Stroffströmen bestehenden Ungleichgewichts findet ein Wärme- und Stoffaustausch statt, der letztlich die gewünschte Auftrennung bedingt. In der Regel befinden sich die trennwirksamen Einbauten in einer Trennkolonne als trennwirksamem Raum R. Insbesondere Rektifikationen sind somit erfindungsgemäß relevante thermische Auftrennungen wenigstens eines Stroms eines (Meth)acrylmonomere enthaltenden Gemischs G. Als trennwirksame Einbauten kommen z. B. Böden (z. B. Dual-Flow Böden, Glockenböden, Ventilböden), Packungen und Füllkörperschüttungen in Betracht. Im Einzelnen kann dabei wie in der DE-A 10332758 beschrieben vorgegangen werden.

Die beim erfindungsgemäßen Verfahren der thermischen Auftrennung in die Rohrleitung der Förderverbindung F3 eingebaute Blende B bildet die Basis dafür, zwischen der Druckseite der Förderpumpe P und dem Ausgang A des Primärraums PR des Umlaufwärmeaustauschers UW ein Druckniveau einzustellen, das oberhalb desjenigen liegt, das an der Rückführstelle RT im trennwirksamen Raum R herrscht. Die Temperatur T^{SII} kann dadurch so gewählt werden, dass der diese Temperatur aufweisende Stoffstrom ST den Umlaufwärmeaustauscher UW an dessen Ausgang A aus dem Primärraum PR im flüssigen Aggregatzustand (d. h. nicht siedend) befindlich verlässt, im flüssigen Aggregatzustand auf die Blende B zuströmt und sich in Strömungsrichtung erst hinter der Blende B befindlich aufgrund des dort herrschenden geringeren Druckniveaus in den Siedezustand übergehend an der Rückführstelle RT in den trennwirksamen Raum R ergießt. Als Umlaufwärmeaustauscher UW wird beim erfindungsgemäßen Verfahren der thermischen Auftrennung vorzugsweise ein Rohrbündelwärmeübertrager eingesetzt (dieser kann einzügig oder mehrzügig ausgebildet sein). Vorzugsweise wird der Stoffstrom ST durch die Wärmeübertragerrohre (das Gesamtinnenvolumen aller Übertragerrohre bildet dann den Primärraum PR) und der fluide Wärmeträger WT durch den die Wärmeübertragerrohre umgebenden Sekundärraum S geführt. Als fluider Wärmeträger WT wird vorzugsweise eine erhöhte Temperatur aufweisender Wasserdampf eingesetzt. Anstelle von Rohrbündelwärmeübertragern können z. B. aber auch Thermoplattenwärmeübertrager verwendet werden. Im Übrigen kann wie in der EP-A 854129, der DE-A 10332758, der DE-A 102008001435 und der DE-A 102008054587 beschrieben vorgegangen werden.

Typische Stromstärken beim erfindungsmäßen Verfahren zum Fördern eines Stroms einer (Meth)acrylmonomere enthaltenden Flüssigkeit F mittels einer Förderpumpe P durch eine Rohrleitung hindurch liegen bei 100 bis 500 000 l/h. Übliche Temperaturen von erfindungsgemäß geförderten Flüssigkeiten F betragen 50 bis 160°C. Der Förderdruck kann sowohl unterhalb als auch oberhalb von Normaldruck liegen. In der Regel liegt der Förderdruck oberhalb von Normaldruck (>1 atm).

Der Verlauf der die Blende B beim erfindungsgemäßen Verfahren eingebaut aufweisenden Rohrleitung kann sowohl horizontal als auch von der Förderpumpe P ausgehend nach oben oder nach unten geneigt verlaufend sein. Ein nach unten geneigter bzw. ein waagrechter Verlauf sind erfindungsgemäß vorteilhaft. Anwendungstechnisch zweckmäßig wird bei einem erfindungsgemäßen Verfahren der kontinuierlichen thermischen Auftrennung wenigstens eines Stroms eines (Meth)acrylmonomere enthaltenden Gemischs G in einer thermischen Trennvorrichtung wie folgt verfahren. Vom Ausgang A aus dem Primärraum PR des Umlaufwärmeaustauschers UW ausgehend verläuft die Förderverbindung F3 zunächst schräg von unten nach oben. Die die Blende B eingebaut aufweisende Rohrleitung ist nachfolgend als Element der Förderverbindung F3 auf dem erreichten erhöhten Niveau waagrecht verlaufend ausgeführt. Die Rückführung der Föderverbindung F3 in den trennwirksamen Raum R erfolgt daran anschließend von diesem erhöhten Niveau nach unten geneigt. Kommt es zu einem Ausfall der Förderpumpe P kommt es bei vorgenanntem Verlauf der Förderverbindung F3 zu einer im Wesentlichen vollständigen Entleerung der erfindungsgemäß betriebenen Förderverbindung F3, was im Fall einer in ihr geförderten (Meth)acrylmonomere enthaltenden Flüssigkeit F aufgrund deren ausgeprägter Neigung zu unerwünschter radikalischer Polymerisaton vorteilhaft ist.

Der Unterschied zwischen T^{SII} und T^{SI} kann bei einem erfindungsgemäßen thermischen Trennverfahren bis zu 30°C oder mehr betragen. Häufig wird er 1 bis 20°C, oder 2 bis 15°C, oder 3 bis 12°C betragen.

Damit umfasst die vorliegende Erfindung insbesondere die nachfolgenden Ausführungsformen:
1. Verfahren zum Fördern eines Stroms einer (Meth)acrylmonomere enthaltenden Flüssigkeit F mittels einer Förderpumpe P durch eine Rohrleitung hindurch, deren offene, für den Strom der Flüssigkeit F durchlässige Querschnittsfläche QF kreisförmig ist und in der an einer in Förderrichtung des Stroms der Flüssigkeit F hinter und/oder vor der Förderpumpe P gelegenen Stelle zum Zweck der Messung und/oder zum Zweck der Begrenzung der Stärke des durch die Rohrleitung geförderten Stroms der Flüssigkeit F eine Blende angebracht ist, die dort eine Teilfläche der offenen kreisförmigen Querschnittsfläche QF schliesst, so dass an dieser Stelle (zum Hindurchströmen für den Strom der Flüssigkeit F) nur noch eine offene Restfläche RF der Querschnittsfläche QF verbleibt, dadurch gekennzeichnet, dass die in der Rohrleitung angebrachte Blende B als Bestandteil der offenen Restfläche RF eine Teilfläche TF der kreisförmigen Querschnittsfläche QF offen lässt, deren Umrisslinie einen Kreisbogen der kreisförmigen Umrisslinie der Querschnittsfläche QF umfasst, welcher im unteren Zehntel der kreisförmigen Umrisslinie der Querschnittsfläche QF liegt.
2. Verfahren gemäß Ausführungsform 1, dadurch gekennzeichnet, dass die Umrisslinie der Teilfläche TF wenigstens 10 % des unteren Zehntels der kreisförmigen Umrisslinie der Querschnittsfläche QF umfasst.
3. Verfahren gemäß Ausführungsform 1, dadurch gekennzeichnet, dass die Umrisslinie der Teilfläche TF wenigstens 30 % des unteren Zehntels der kreisförmigen Umrisslinie der Querschnittsfläche QF umfasst.
4. Verfahren gemäß Ausführungsform 1, dadurch gekennzeichnet, dass die Umrisslinie der Teilfläche TF wenigstens 40 % des unteren Zehntels der kreisförmigen Umrisslinie der Querschnittsfläche QF umfasst.
5. Verfahren gemäß Ausführungsform 1, dadurch gekennzeichnet, dass die Umrisslinie der Teilfläche TF wenigstens 60 % des unteren Zehntels der kreisförmigen Umrisslinie der Querschnittsfläche QF umfasst.
6. Verfahren gemäß Ausführungsform 1, dadurch gekennzeichnet, dass die Umrisslinie der Teilfläche TF wenigstens 80 % des unteren Zehntels der kreisförmigen Umrisslinie der Querschnittsfläche QF umfasst.
7. Verfahren gemäß Ausführungsform 1, dadurch gekennzeichnet, dass die Umrisslinie der Teilfläche TF das gesamte untere Zehntel der kreisförmigen Umrisslinie der Querschnittsfläche QF umfasst.
8. Verfahren gemäß Ausführungsform 1, dadurch gekennzeichnet, dass die Umrisslinie der Teilfläche TF einen Kreisbogen der kreisförmigen Umrisslinie der Querschnittsfläche QF umfasst, welcher im unteren Zwanzigstel der kreisförmigen Umrisslinie der Querschnittsfläche QF liegt.
9. Verfahren gemäß Ausführungsform 8, dadurch gekennzeichnet, dass die Umrisslinie der Teilfläche TF wenigstens 10 % des unteren Zwanzigstels der kreisförmigen Umrisslinie der Querschnittsfläche QF umfasst.
10. Verfahren gemäß Ausführungsform 8, dadurch gekennzeichnet, dass die Umrisslinie der Teilfläche TF wenigstens 30 % des unteren Zwanzigstels der kreisförmigen Umrisslinie der Querschnittsfläche QF umfasst.
11. Verfahren gemäß Ausführungsform 8, dadurch gekennzeichnet, dass die Umrisslinie der Teilfläche TF wenigstens 50 % oder wenigstens 70 % des unteren Zwanzigstels der kreisförmigen Umrisslinie der Querschnittsfläche QF umfasst.
12. Verfahren gemäß Ausführungsform 8, dadurch gekennzeichnet, dass die Umrisslinie der Teilfläche TF das gesamte untere Zwanzigstel der kreisförmigen Umrisslinie der Querschnittsfläche QF umfasst.
13. Verfahren gemäß einer der Ausführungsformen 1 bis 12, dadurch gekennzeichnet, dass die Umrisslinie der Teilfläche TF einen Kreisbogen der kreisförmigen Umrisslinie der Querschnittsfläche QF umfasst, auf welchem der am tiefsten gelegene Punkt TP der kreisförmigen Umrisslinie der Querschnittsfläche QF liegt.
14. Verfahren gemäß einer der Ausführungsformen 1 bis 13, dadurch gekennzeichnet, dass die offene Restfläche RF 10 bis 90 % der Querschnittsfläche QF beträgt.
15. Verfahren gemäß einer der Ausführungsformen 1 bis 13, dadurch gekennzeichnet, dass die offene Restfläche RF 20 bis 80 % der Querschnittsfläche QF beträgt.
16. Verfahren gemäß einer der Ausführungsformen 1 bis 13, dadurch gekennzeichnet, dass die offene Restfläche RF 30 bis 70 % der Querschnittsfläche QF beträgt.
17. Verfahren gemäß einer der Ausführungsformen 1 bis 16, dadurch gekennzeichnet, dass die Teilfläche TF 5 bis 95 % der offenen Restfläche RF beträgt.
18. Verfahren gemäß einer der Ausführungsformen 1 bis 16, dadurch gekennzeichnet, dass die Teilfläche TF 20 bis 80 % der offenen Restfläche RF beträgt.
19. Verfahren gemäß einer der Ausführungsformen 1 bis 16, dadurch gekennzeichnet, dass die Teilfläche TF 30 bis 70 % der offenen Restfläche RF beträgt.
20. Verfahren gemäß einer der Ausführungsformen 1 bis 16, dadurch gekennzeichnet, dass die Teilfläche TF mit der offenen Restfläche RF identisch ist.
21. Verfahren gemäß einer der Ausführungsformen 1 bis 20, dadurch gekennzeichnet, dass die Flüssigkeit F wenigstens ein (Meth)acrylmonomeres aus der Gruppe bestehend aus Acrolein, Methacrolein, Acrylsäure, Methacrylsäure, Hydroxyethylacrylat, Hydroxyethylmethacrylat, Hydroxypropylacrylat, Hydroxypropylmethacrylat, Glycydydylacrylat, Glycydylmethacrylat, Methylacrylat, Methylmethacrylat, n-Butylacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, 2-Propylheptylacrylat, N,N-Diethylaminoethylacrylat und N,N-Dimethylaminoethylmethacrylat enthält.
22. Verfahren gemäß einer der Ausführungsformen 1 bis 20, dadurch gekennzeichnet, dass die Flüssigkeit F wenigstens eines der Monomeren Acrolein, Methacrolein, Acrylsäure, Methacrylsäure, Ester aus Acrylsäure und einem 1 bis 12 C-Atome aufweisenden Alkohol und Ester aus Methacrylsäure und einem 1 bis 12 C-Atome aufweisenden Alkohol enthält.
23. Verfahren gemäß einer der Ausführungsformen 1 bis 22, dadurch gekennzeichnet, dass der Gehalt der Flüssigkeit F an (Meth)acrylmonomeren ≥ 5 Gew.-% beträgt.
24. Verfahren gemäß einer der Ausführungsformen 1 bis 22, dadurch gekennzeichnet, dass der Gehalt der Flüssigkeit F an (Meth)acrylmonomeren ≥ 20 Gew.-% beträgt.
25. Verfahren gemäß einer der Ausführungsformen 1 bis 22, dadurch gekennzeichnet, dass der Gehalt der Flüssigkeit F an (Meth)acrylmonomeren ≥ 40 Gew.-% beträgt.
26. Verfahren gemäß einer der Ausführungsformen 1 bis 22, dadurch gekennzeichnet, dass der Gehalt der Flüssigkeit F an (Meth)acrylmonomeren ≥ 60 Gew.-% beträgt.
27. Verfahren gemäß einer der Ausführungsformen 1 bis 22, dadurch gekennzeichnet, dass der Gehalt der Flüssigkeit F an (Meth)acrylmonomeren ≥ 80 Gew.-% beträgt.
28. Verfahren gemäß einer der Ausführungsformen 1 bis 27, dadurch gekennzeichnet, dass die Flüssigkeit F eine Lösung ist.
29. Verfahren gemäß einer der Ausführungsformen 1 bis 28, dadurch gekennzeichnet, dass die Temperatur der Flüssigkeit F 50 bis 160°C beträgt.
30. Verfahren gemäß einer der Ausführungsformen 1 bis 29, dadurch gekennzeichnet, dass der zur kreisförmigen Querschnittsfläche QF gehörige Durchmesser 1 bis 100 cm, oder 10 bis 90 cm, oder 30 bis 70 cm beträgt.
31. Verfahren gemäß einer der Ausführungsformen 1 bis 30, dadurch gekennzeichnet, dass die Stromstärke der durch die Rohrleitung geförderten Flüssigkeit F 100 bis 500 000 l/h beträgt.
32. Verfahren gemäß einer der Ausführungsformen 1 bis 31, dadurch gekennzeichnet, dass der Druck in der geförderten Flüssigkeit F oberhalb von 1 atm liegt.
33. Verfahren der kontinuierlichen thermischen Auftrennung wenigstens eines Stroms eines (Meth)acrylmonomere enthaltenden Gemischs G in einer thermischen Trennvorrichtung, die
   - eine Förderpumpe P, die eine Saugseite und eine Druckseite aufweist,
   - einen indirekten Umlaufwärmeaustauscher UW, der einen Sekundärraum S und einen von dem Sekundärraum S durch die materielle Trennwand T getrennten Primärraum PR umfasst, der einen Eingang E und einen Ausgang A aufweist,
   - einen eine Zuführstelle ZU und eine Rückführstelle RT aufweisenden trennwirksamen Raum R mit oder ohne trennwirksame Einbauten, in den an der Zuführstelle ZU kontinuierlich der Strom des Gemischs G hineingeführt wird,
   - eine erste Förderverbindung F1, die vom trennwirksamen Raum R zur Saugseite der Förderpumpe P führt,
   - eine zweite Förderverbindung F2, die von der Druckseite der Förderpumpe P zum Eingang E in den Primärraum PR des Umlaufwärmeaustauschers UW führt,
   - eine dritte Förderverbindung F3, die vom Ausgang A aus dem Primärraum PR des Umlaufwärmeaustauschers UW zu der Rückführstelle RT in den trennwirksamen Raum R zurückführt und eine Rohrleitung RL mit einer offenen kreisförmigen Querschnittsfläche QF aufweist, und
   - eine Blende B, die in der Rohrleitung RL der Förderverbindung F3 an einer zwischen dem Ausgang A aus dem Primärraum PR des Umlaufwärmeaustauschers UW und der Rückführstelle RT in den trennwirksamen Raum R gelegenen Stelle angebracht ist und dort eine Teilfläche der offenen kreisförmigen Querschnittsfläche QF der Rohrleitung RL schließt, so dass an dieser Stelle nur noch eine offene Restfläche RF der Querschnittsfläche QF in der Rohrleitung RL der Förderverbindung F3 verbleibt,
   umfasst, und bei dem wenigstens ein Teil der für die thermische Auftrennung des Gemischs G im trennwirksamen Raum R erforderlichen Energie dadurch in denselben eingebracht wird, dass
   - die Förderpumpe P aus dem trennwirksamen Raum R kontinuierlich einen flüssigen, die Temperatur T^{SI} aufweisenden, (Meth)acrylmonomere enthaltenden Stoffstrom ST ansaugt,
   - die Förderpumpe P den von ihr angesaugten Stoffstrom ST durch die Förderverbindung F2, den Primärraum PR des Umlaufwärmeaustauschers UW sowie die Förderverbindung F3 hindurch an der Rückführstelle RT in den trennwirksamen Raum R zurückpumpt, und
   - gleichzeitig durch den Sekundärraum S ein fluider Wärmeträger WT, dessen Temperatur T^{WT}, mit der er in den Sekundärraum S hineingeführt wird, größer als die Temperatur T^{SE} des Stoffstroms ST am Eingang E in den Primärraum PR ist, so hindurchgeführt wird, dass der Stoffstrom ST aus dem Ausgang A des Primärraums PR des Umlaufwärmeaustauschers UW flüssig auf die Restfläche RF zuströmt und die Restfläche RF mit der Temperatur T^{SII} > T^{SI} durchströmt,
   das dadurch gekennzeichnet ist, dass die in der Rohrleitung RL der Förderverbindung F3 eingebaute Blende B als Bestandteil der offenen Restfläche RF eine Teilfläche TF der kreisförmigen Querschnittsfläche QF offen lässt, deren Umrisslinie einen Kreisbogen der kreisförmigen Umrisslinie der Querschnittsfläche QF umfasst, welcher im unteren Zehntel der kreisförmigen Umrisslinie der Querschnittsfläche QF liegt.
34. Verfahren gemäß Ausführungsform 33, dadurch gekennzeichnet, dass der trennwirksame Raum R wenigstens einen Auslass AU1, aus dem ein (Meth)acrylmonomere angereichert enthaltender Stoffstrom 1, und wenigstens einen Auslass AU2, aus dem ein (Meth)acrylmonomere abgereichert enthaltender Stoffstrom 2 herausgeführt wird, aufweist.
35. Verfahren gemäß Ausführungsform 33 oder 34, dadurch gekennzeichnet, dass die Förderung des flüssigen Stoffstroms ST durch die Rohrleitung RL ein Verfahren gemäß einer der Ausführungsformen 1 bis 32 ist, wobei der flüssige Stoffstrom ST der gemäß den Ausführungsformen 1 bis 32 zu fördernde Strom der Flüssigkeit F ist.

### Beispiel und Vergleichsbeispiel

Verfahren der kontinuierlichen thermischen Auftrennung eines Stroms eines Acrylsäure enthaltenden Gemischs G in einer thermischen Trennvorrichtung

Das flüssige Gemisch G wies nachfolgende Gehalte auf:
4,49 Gew.-% Acrylsäure,
0,0253 Gew.-% Essigsäure,
0,0099 Gew.-% Wasser,
55,77 Gew.-% Diphyl,
37,85 Gew.-% Dimethylphthalat,
0,0003 Gew.-% Ameisensäure,
0,0025 Gew.-% Acrolein,
0,0007 Gew.-% Propionsäure,
0,0023 Gew.-% Furfurale,
0,0001 Gew.-% Allylacrylat,
0,0291 Gew.-% Benzaldehyd,
0,01374 Gew.-% Maleinsäureanhydrid,
0,366 Gew.-% Benzoesäure,
0,966 Gew.-% Diacrylsäure,
0,280 Gew.-% Phenothiazin, und
0,0001 Gew.-% molekularer Sauerstoff.

Seine Temperatur betrug 152,4°C. Es wurde dem Sumpfbereich einer Absorptionskolonne entnommen. Das Absorptionsverfahren verfolgte den Zweck der Abtrennung von Acrylsäure aus dem Produktgasgemisch einer heterogen katalysierten partiellen Gasphasenoxidation von Propen zu Acrylsäure, wie es in der DE-A-10336386 beschrieben ist.

8902 kg/h des flüssigen Gemischs G wurden einer thermischen Trennvorrichtung zugeführt, die eine Trennkolonne als trennwirksamer Raum R und einen Umlaufwärmeaustauscher UW sowie eine Förderpumpe P umfasste.

Der Umlaufwärmeaustauscher UW war ein Achtstromrohrbündelwärmeüberträger, der 704 Wärmeübertragerrohre enthielt. Der Innendurchmesser der Rohre betrug einheitlich 21 mm, bei einer Wanddicke von 2 mm und einer Rohrlänge von 2500 mm.
Das Fertigungsmaterial war DIN Werkstoff 1.4571. Der Innendurchmesser des kreiszylindrischen Wärmeübertragers betrug 1100 mm. Als Wärmeträger wurden dem die Wärmeübertragerrohre umgebenden Sekundärraum des Rohrbündelwärmeaustauschers 1800 kg/h Sattwasserdampf (29 bar, 231°C) zugeführt. Mittels 7 kreisförmigen Umlenkblechen (das Verhältnis von freiem Querschnitt zu verschlossenem Querschnitt betrug jeweils 3 : 8) wurde der Wasserdampfstrom im Rohrbündelwärmeübertrager um die Übertragerrohre herumgeleitet. Das sich im Wärmeübertrager ausbildende Wasserdampfkondensat wurde mit einer Temperatur von 200°C aus dem Wärmeübertrager herausgeführt.

Die den Zwangsumlauf bewerkstelligende Förderpumpe P war eine Radialkreiselpumpe mit einem geschlossenen Laufrad der Fa. Sulzer vom Typ ZE 200/400. Als Sperrflüssigkeit wurde eine Mischung aus 50 Gew.-% Glykol und 50 Gew.-% Wasser verwendet.

Die Trennkolonne hatte einen zylindrischen Querschnitt mit einem Innendurchmesser von 2200 mm. Die Höhe des zylindrischen Teils betrug 7402 mm.
Fertigungsmaterial war DIN Werkstoff 1.4571, die Wandstärke betrug 12 mm. Der Innendurchmesser des oberen Austrittsstutzens betrug 900 mm, der Durchmesser des unteren Austrittstutzens war 400 mm. Die Trennkolonne wies keine trennwirksamen Einbauten auf. Der obere Austrittstutzen war auf einer Länge von 558 mm in die Trennkolonne hineingeführt. Um diesen in die Kolonne hineingeführten Stutzen herum war vom oberen Ende der Trennkolonne nach unten ragend zusätzlich ein ringförmiger Kragen angebracht, dessen Kragenlänge 500 mm betrug. Der Kopfdruck der Trennkolonne war auf 85 mbar eingestellt. Die Trennkolonne wurde ohne Rücklaufflüssigkeit betrieben. Die Trennkolonne wurde standgeregelt betrieben. Die maximale Standhöhe der am unteren Ende der Trennkolonne aufgelaufenen (noch Acrylsäure enthaltenden) Flüssigkeit war 1932 mm und die minimale Standhöhe war 900 mm. Die Zufuhr des flüssigen Gemischs G in die Trennkolonne erfolgte in dem entsprechender Weise getaktet. Durch den Kopf des oberen Austrittstutzens der Trennkolonne wurden 8642 kg/h an in der Kolonne gebildeten Brüden mit einer Temperatur von 180°C herausgeführt.

Die Gehalte dieses Brüdenstroms waren:

| | |
|---|---|
| 4,346 | Gew.-% Acrylsäure, |
| 0,0260 | Gew.-% Essigsäure, |
| 0,0102 | Gew.-% Wasser, |
| 56,46 | Gew.-% Diphyl, |
| 37,279 | Gew.-% Dimethylphthalat, |
| 0,0003 | Gew.-% Ameisensäure, |
| 0,0025 | Gew.-% Acrolein, |
| 0,0007 | Gew.-% Propionsäure, |
| 0,0024 | Gew.-% Furfurale, |
| 0,0001 | Gew.-% Allylacrylat, |
| 0,0299 | Gew.-% Benzaldehyd, |
| 0,141 | Gew.-% Maleinsäureanhydrid, |
| 0,369 | Gew.-% Benzoesäure, |
| 1,244 | Gew.-% Diacrylsäure, |
| 0,0004 | Gew.-% Glyoxal und |
| 0,0001 | Gew.-% molekularer Sauerstoff. |

Mit der Förderpumpe P wurde aus dem unteren Austrittstutzen der Trennkolonne mit der Temperatur T^{SI} = 180°C ein flüssiger (noch Acrylsäure enthaltender) Stoffstrom S von 204176 kg/h angesaugt. Ein Teilmengenstrom desselben wurde ausgeschleust und seiner Verbrennung zugeführt. Der verbliebene flüssige Reststrom von 203916 kg/h wurde durch die Wärmeübertragerrohre des Umlaufwärmeaustauschers UW gepumpt. Der Reststrom strömte mit einer Temperatur von 189°C flüssig aus den Wärmeübertragerrohren (dem Primärraum PR des Umlaufwärmeaustauschers UW). Durch eine Rohrleitung, die eine offene Querschnittsfläche von 196250 mm² und ein Innenvolumen von 1,8 m³ aufwies, hindurch, strömte der 189°C aufweisende flüssige Reststrom in Richtung Trennkolonne. Nach etwa zwei Dritteln der Gesamtlänge der Rohrleitung war in derselben eine Lochblende angebracht. Die Querschnittsfläche der Lochöffnung betrug 49063 mm². Auf einer Höhe von etwa 3563 mm (von unten) erfolgte die Rückführung des überhitzten Reststroms (dessen Druck am Ausgang aus den Wärmeübertragerrohren 4 bar betrug) in die Trennkolonne. Auf derselben Höhe erfolgte die Zufuhr des Gemischs G in die Trennkolonne.

Nach einer Betriebsdauer von 2 Monaten, 3 Wochen und 4 Tagen musste das Verfahren der thermischen Auftrennung unterbrochen werden, da die Lochöffnung der Lochblende verschlossen war.

Wurde die Lochblende durch eine Blende B gemäß Figur 3 ersetzt, deren offene Restfläche ebenfalls 49063 mm² betrug, war auch nach einer Betriebsdauer von Monaten noch kein Verschluss der Blende Ausgebildet.

## Patentansprüche

1. Verfahren zum Fördern eines Stroms einer (Meth)acrylmonomere enthaltenden Flüssigkeit F mittels einer Förderpumpe P durch eine Rohrleitung hindurch, deren offene, für den Strom der Flüssigkeit F durchlässige Querschnittsfläche QF kreisförmig ist und in der an einer in Förderrichtung des Stroms der Flüssigkeit F hinter und/oder vor der Förderpumpe P gelegenen Stelle zum Zweck der Messung und/oder zum Zweck der Begrenzung der Stärke des durch die Rohrleitung geförderten Stroms der Flüssigkeit F eine Blende B angebracht ist, die dort eine Teilfläche der offenen kreisförmigen Querschnittsfläche QF schließt, so dass an dieser Stelle nur noch eine offene Restfläche RF der Querschnittsfläche QF verbleibt, **dadurch gekennzeichnet, dass** die in der Rohrleitung angebrachte Blende B als Bestandteil der offenen Restfläche RF eine Teilfläche TF der kreisförmigen Querschnittsfläche QF offen lässt, deren Umrisslinie einen Kreisbogen der kreisförmigen Umrisslinie der Querschnittsfläche QF umfasst, welcher im unteren Zehntel der kreisförmigen Umrisslinie der Querschnittsfläche QF liegt.

2. Verfahren der kontinuierlichen thermischen Auftrennung wenigstens eines Stroms eines (Meth)acrylmonomere enthaltenden Gemischs G in einer thermischen Trennvorrichtung, die
- eine Förderpumpe P, die eine Saugseite und eine Druckseite aufweist,
- einen indirekten Umlaufwärmeaustauscher UW, der einen Sekundärraum S und einen von dem Sekundärraum S durch eine materielle Trennwand T getrennten Primärraum PR umfasst, der einen Eingang E und einen Ausgang A aufweist,
- einen eine Zuführstelle ZU und eine Rückführstelle RT aufweisenden trennwirksamen Raum R mit oder ohne trennwirksame Einbauten, in den an der Zuführstelle ZU kontinuierlich der Strom des Gemischs G hineingeführt wird,
- eine erste Förderverbindung F1, die vom trennwirksamen Raum R zur Saugseite der Förderpumpe P führt,
- eine zweite Förderverbindung F2, die von der Druckseite der Förderpumpe P zum Eingang E in den Primärraum PR des Umlaufwärmeaustauschers UW führt,
- eine dritte Förderverbindung F3, die vom Ausgang A aus dem Primärraum PR des Umlaufwärmeaustauschers UW zu der Rückführstelle RT in den trennwirksamen Raum R zurückführt und eine Rohrleitung RL mit einer offenen kreisförmigen Querschnittsfläche QF aufweist, und
- eine Blende B, die in der Rohrleitung RL der Förderverbindung F3 an einer zwischen dem Ausgang A aus dem Primärraum PR des Umlaufwärmeaustauschers UW und der Rückführstelle RT in den trennwirksamen Raum R gelegenen Stelle angebracht ist und dort eine Teilfläche der offenen kreisförmigen Querschnittsfläche QF der Rohrleitung RL schließt, so dass an dieser Stelle nur noch eine offene Restfläche RF der Querschnittsfläche QF in der Rohrleitung RL der Förderverbindung F3 verbleibt,
umfasst, und bei dem wenigstens ein Teil der für die thermische Auftrennung des Gemischs G im trennwirksamen Raum R erforderlichen Energie dadurch in denselben eingebracht wird, dass
- die Förderpumpe P aus dem trennwirksamen Raum R kontinuierlich einen flüssigen, die Temperatur T^{SI} aufweisenden, (Meth)acrylmonomere enthaltenden Stoffstrom ST ansaugt,
- die Förderpumpe P den von ihr angesaugten Stoffstrom ST durch die Förderverbindung F2, den Primärraum PR des Umlaufwärmeaustauschers UW sowie die Förderverbindung F3 hindurch an der Rückführstelle RT in den trennwirksamen Raum R zurückpumpt, und
- gleichzeitig durch den Sekundärraum S ein fluider Wärmeträger WT, dessen Temperatur T^{WT}, mit der er in den Sekundärraum S hineingeführt wird, größer als die Temperatur T^{SE} des Stoffstroms ST am Eingang E in den Primärraum PR ist, so hindurchgeführt wird, dass der Stoffstrom ST aus dem Ausgang A des Primärraums PR des Umlaufwärmeaustauschers UW flüssig auf die Restfläche RF zuströmt und die Restfläche RF mit der Temperatur T^{SII} > T^{SI} durchströmt,
**dadurch gekennzeichnet, dass** die in der Rohrleitung RL der Förderverbindung F3 eingebaute Blende B als Bestandteil der offenen Restfläche RF eine Teilfläche TF der kreisförmigen Querschnittsfläche QF offen lässt, deren Umrisslinie einen Kreisbogen der kreisförmigen Umrisslinie der Querschnittsfläche QF umfasst, welcher im unteren Zehntel der kreisförmigen Umrisslinie der Querschnittsfläche QF liegt.

## Claims

1. A process for conveying a stream of a liquid F comprising (meth)acrylic monomers by means of a delivery pump P through a pipeline whose open cross-sectional area QF pervious to the stream of liquid F is circular and in which is mounted, at a point beyond and/or upstream of the delivery pump P in conveying direction of the stream of liquid F, for the purpose of measurement and/or for the purpose of limiting the flow rate of the stream of liquid F conveyed through the pipeline, a restrictor B which closes an area portion of the open circular cross-sectional area QF there, such that only an open residual area RF of the cross-sectional area QF remains at this point, wherein the restrictor B mounted in the pipeline leaves open, as a constituent of the open residual area RF, an area portion TF of the circular cross-sectional area QF, the outline of which comprises a circular arc of the circular outline of the cross-sectional area QF, said circular arc being within the lower tenth of the circular outline of the cross-sectional area QF.

2. A process for continuously thermally separating at least one stream of a mixture G comprising (meth)acrylic monomers in a thermal separating apparatus which comprises
- a delivery pump P which has a suction side and a pressure side,
- an indirect circulation heat exchanger UW which comprises a secondary space S and a primary space PR which is separated from the secondary space S by a material dividing wall T and has an inlet E and an outlet A,
- a separating space R which has a feed point ZU and a return point RT and has or does not have separating internals, into which the stream of the mixture G is conducted continuously at the feed point ZU,
- a first delivery connection F1 which leads from the separating space R to the suction side of the delivery pump P,
- a second delivery connection F2 which leads from the pressure side of the delivery pump P to the inlet E into the primary space PR of the circulation heat exchanger UW,
- a third delivery connection F3 which leads from the outlet A from the primary space PR of the circulation heat exchanger UW to the return point RT into the separating space R, and has a pipeline RL with an open circular cross-sectional area QF, and
- a restrictor B which is mounted in the pipeline RL of the delivery connection F3 at a site between the outlet A from the primary space PR of the circulation heat exchanger UW and the return point RT into the separating space R, and closes an area portion of the open circular cross-sectional area QF of the pipeline RL there, such that only an open residual area RF of the cross-sectional area QF remains at this point in the pipeline RL of the delivery connection F3,
and in which at least a portion of the energy required for the thermal separation of the mixture G in the separating space R is introduced into the latter by virtue of
- the delivery pump P continuously sucking a liquid stream ST which has the temperature T^{SI} and comprises (meth)acrylic monomers out of the separating space R,
- the delivery pump P pumping the stream ST that it has sucked in through the delivery connection F2, the primary space PR of the circulation heat exchanger UW and the delivery connection F3 back into the separating space R at the return point RT, and
- at the same time, a fluid heat carrier WT whose temperature T^{WT} with which it is conducted into the secondary space S is greater than the temperature T^{SE} of the stream ST at the inlet E into the primary space PR being conducted through the secondary space S such that the stream ST flows out of the outlet A of the primary space PR of the circulation heat exchanger UW in liquid form toward the residual area RF and flows through the residual area RF with the temperature T^{SII} > T^{SI},
wherein the restrictor B installed in the pipeline RL of the delivery connection F3, as a constituent of the open residual area RF, leaves open an area portion TF of the circular cross-sectional area QF whose outline comprises a circular arc of the circular outline of the cross-sectional area QF, said circular arc being within the lower tenth of the circular outline of the cross-sectional area QF.

## Revendications

1. Procédé pour le transport d'un courant d'un liquide F contenant des monomères (méth)acryliques au moyen d'une pompe de refoulement P au travers d'un conduit tubulaire dont la surface de section transversale ouverte QF par laquelle peut passer le courant du liquide F est circulaire et dans laquelle en un point situé après et/ou avant la pompe de refoulement P dans le sens de transport du courant du liquide F, dans le but de mesure et/ou dans le but de la limitation de la force du courant du liquide F transporté au travers du conduit tubulaire, est placé un diaphragme B qui y obture une surface partielle de la surface de la section transversale circulaire ouverte QF, de sorte qu'en ce point il ne reste plus qu'une surface restante ouverte RF de la surface de la section transversale QF, **caractérisé en ce que** le diaphragme B placé dans le conduit tubulaire laisse ouvert en tant que composante de la surface restante ouverte RF une surface partielle TF de la surface de la section transversale circulaire QF, dont la ligne de contour comprend un arc de cercle de la ligne de contour circulaire de la surface de la section transversale QF, qui se trouve dans le dixième inférieur de la ligne de contour circulaire de la surface de la section transversale QF.

2. Procédé de fractionnement thermique continu d'au moins un courant d'un mélange G contenant des monomères (méth)acryliques dans un dispositif de fractionnement qui comprend
- une pompe de refoulement P qui comporte un côté aspiration et un côté refoulement,
- un échangeur thermique indirect à circulation UW, qui comprend une chambre secondaire S et une chambre primaire PR séparée de la chambre secondaire S par une paroi de séparation matérielle T, qui comporte une entrée E et une sortie A,
- une chambre R à action de séparation, comportant un point d'alimentation ZU et un point de remise en circulation RT, avec ou sans inserts actifs pour la séparation, dans laquelle le courant du mélange G est introduit en continu au niveau du point d'alimentation ZU,
- un premier raccord de circulation F1, qui conduit de la chambre R à action de séparation au côté aspiration de la pompe de refoulement P,
- un deuxième raccord de circulation F2, qui conduit du côté refoulement de la pompe de refoulement P à l'entrée E dans la chambre primaire PR de l'échangeur thermique à circulation UW,
- un troisième raccord de circulation F3, qui renvoie de la sortie A de la chambre primaire PR de l'échangeur thermique à circulation UW au point de remise en circulation RT dans la chambre à action de séparation R et comporte un conduit tubulaire RL ayant une surface de section transversale circulaire ouverte QF, et
- un diaphragme B qui est placé dans le conduit tubulaire RL du raccord de circulation F3 en un point situé entre la sortie A de la chambre primaire PR de l'échangeur thermique à circulation UW et le point de remise en circulation RT dans la chambre R à action de séparation et y obture une surface partielle de la surface de la section transversale circulaire ouverte QF du conduit tubulaire RL, de sorte qu'en ce point il ne reste plus qu'une surface restante ouverte RF de la surface de la section transversale QF dans le conduit tubulaire RL du raccord de circulation F3,
et dans lequel au moins une partie de l'énergie requise pour le fractionnement thermique du mélange G dans la chambre R à action de séparation est introduite dans celle-ci par le fait que
- la pompe de refoulement P aspire en continu de la chambre R à action de séparation un courant de substances ST contenant des monomères (méth)acryliques, présentant la température T^{SI},
- la pompe de refoulement P refoule le courant de substances ST aspiré par celle-ci, via le raccord de circulation F2, la chambre primaire PR de l'échangeur thermique à circulation UW ainsi que le raccord de circulation F3 au niveau du point de remise en circulation RT dans la chambre R à action de séparation, et
- en même temps on fait passer dans la chambre secondaire S un agent caloporteur fluide WT, dont la température T^{WT} à laquelle il est introduit dans la chambre secondaire S, est supérieure à la température T^{SE} du courant de substances ST à l'entrée E dans la chambre primaire PR, de sorte que le courant de substances ST s'écoule à l'état liquide de la sortie A de la chambre primaire PR de l'échangeur thermique à circulation UW sur la surface restante RF et traverse la surface restante RF à la température T^{SII} > T^{SI},
**caractérisé en ce que** le diaphragme B inséré dans le conduit tubulaire RL du raccord de circulation F3 en tant que composante de la surface restante ouverte RF laisse ouverte une surface partielle TF de la surface de la section transversale circulaire QF, dont la ligne de contour comprend un arc de cercle de la ligne de contour circulaire de la surface de la section transversale QF, qui se trouve dans le dixième inférieur de la ligne de contour circulaire de la surface de la section transversale QF.
